# EUROPEAN PATENT SPECIFICATION

(11) **EP 3 449 403 B1**
(45) Date of publication and mention of the grant of the patent: **20.11.2024**
(21) Application number: 17722701.4
(22) Date of filing: 25.04.2017
(51) Int. Cl.: G16H 15/00, G16H 30/20, G16H 30/40

(54) **METHOD AND SYSTEM FOR RADIOLOGY REPORTING**
VERFAHREN UND SYSTEM ZUR RADIOLOGISCHEN BERICHTERSTATTUNG
PROCÉDÉ ET SYSTÈME D'ÉMISSION DE RAPPORT DE RADIOLOGIE

(30) Priority: 26.04.2016 BE 201605285
(43) Date of publication of application: 06.03.2019
(73) Proprietor: Grain IP, 9300 Aalst (BE)
(72) Inventor: VAN HOE, Lieven, 9300 Aalst (BE)
(74) Representative: De Clercq & Partners
(86) International application number: PCT/EP2017/059763
(87) International publication number: WO 2017/186699

(56) References cited:
- WO-A2-2012/037049
- WO-A2-2012/071571
- US-A1- 2005 226 405
- US-A1- 2012 330 876
- ANONYMOUS: "EHR Systems - Two monitors are better than one", MEDICALSOFTWARESOLUTIONS.NET, 28 August 2013 (2013-08-28), pages 1 - 1, XP093010492, Retrieved from the Internet <URL:http://medicalsoftwaresolutions.net/ehr-systems-two-monitors-better-one/> [retrieved on 20221222]

## Description

### Field of the invention

The invention generally relates to generating a report of the analysis of a medical image, and in particular generating a report for radiological image files.

### Background to the invention

Medical imaging techniques are commonly used for diagnosis and/or follow-up in clinical medicine. Examples of medical imaging techniques include X-Ray, CT (computed tomography), MRI (magnetic resonance imaging), mammography, ultrasonography, etc.

Usually, a medical professional (typically a radiologist) analyses the images and dictates a medical imaging report. Methods and systems for generating a report are known in the art, for instance, WO 2012/037049 describes a teleradiology system having a report generation system that is template driven and provides access to macros specific to a study as well as previously dictated entries for particular fields to allow reuse of standard text in a contextual manner. WO 2012/071571 discloses a method for creating a radiological report from radiological images, including a fitting of a structural template with a radiological image and using landmark data and pathological data to populate empty fields of the report template. US 2012/0330876 describes a method for radiologists to report both normal and abnormal findings using frequently-repeated phrases. US 2005/0226405 discloses a method for creating a medical report that provides a link list to whole images captured in a time-series manner.

Traditionally, free dictation is used, whereby the radiologist dictates his observations in a self-chosen format, style, order, and language. With speech recognition, the radiologist is able to see a written transcript of his spoken report, and to make changes if required.

While dictating, the radiologist views the images. With modern techniques like CT and MRI, large sets of images are obtained, and radiologists typically use a computer mouse to scroll through the images. In a classic set-up, the radiologist uses one hand to operate the computer mouse (to scroll through the set of images), and the other hand to hold a dedication tool that records the words spoken by the radiologist (a "dictaphone").

Disadvantages of free dictation are well recognised and include a lack of standardisation and a high variability in report content, structure and quality.

In an attempt to overcome these limitations, structured reporting has been proposed as an alternative to free dictation.

Structured reporting has three essential characteristics: (1) the report has a structured format, with paragraphs and headings, (2) the report has a consistent organisation (e.g., each of the relevant anatomic areas are described in the report), and (3) a standard language is used. Using reports with a structured format and a consistent organization is sometimes called "itemised reporting" or "standardised reporting".

In practice, in order to make a standardised / itemised report, the radiologists starts with a predefined reporting template (suitable for the type of exam that has to be reported) and makes changes where needed. The template may be a normal report, a report where some findings have to be added, or a checklist that guides the radiologist. The radiologist analyses the images and changes the template report where needed. Usually, a two-monitor configuration is used. For example, images can be viewed on the right monitor, and the report is shown (and can be edited) on the left monitor (see below).

Structured reporting (SR) (and also standardized reporting) is highly controversial in radiology. While most experts acknowledge that making structured / standardized reports would be beneficial for quality of care, acceptance of systems proposed has been slow or even non-existent.

The reason is that SR systems tend to distract radiologists from their main task: looking at images and simultaneously dictating a report. From the point of view of the reporting radiologist, free dictation is usually preferred over itemised reporting, because, in free dictation, the cognitive process of scrolling through images, interpreting images, and outputting the interpretation as dictated text is highly effective. In itemised reporting, on the other hand, the radiologist has to look away from the images every time an item has to be checked and/or edited in the report template; as such the radiologist is forced to look away from the images and cognitive workflow is interrupted. Switching attention between multiple- screens can induce oculomotor distress, such as headaches, eyestrain, blurred vision and general discomfort over time. The present invention aims to overcome the problems of the art.

### Brief Summary of the Invention

The present invention relates to method for assisting a user in generating an itemised medical report from at least one medical image in accordance with claim 1. The at least one medical image is displayed in a display area of single computer display unit. In the same display area, a sub-region containing a checklist specific to the one or more images is displayed. Items from the checklist can be de-selected. This checklist comprises a list of selectable items, each item representing an organ, structure, or abnormality that has to be checked by the user. Linked to each item in the checklist is a default statement that is a pre-prepared statement indicative of the normality of the item. The default statement is not displayed by default as part of the checklist. The user can select an item from the checklist for providing comments by dictation thereon responsive to an observation in the radiological image. At the end of the image analysis, an editable itemised medical report is generated containing each item of the checklist as a heading and either the dictated comment or the default statement associated with the item as an observation. The editable itemised medical report is rapidly and accurately generated. Information is confined to a single screen reducing the look away time for the user. The user experiences reduced oculomotor distress and improved concentration.

Accordingly, presently claimed in claim 1 is a method for assisting a user in generating an itemised medical report from at least one medical image (410), comprising the steps of:
- generating for display and displaying in a display area (400) of single computer display unit (100) at least one of the medical images (410);
- generating for display and displaying in the same display area (400), a sub-region (200) containing a checklist (205) specific to the one or more images, comprising a list of selectable items (210), wherein each item (210a', 210a", 210a'", 210a'", 210a'") in the checklist is linked to a default statement (510a' , 510a", 510a‴, 510aʺʺ) which default statement is by default not displayed in the same display area (400) and is a pre-prepared statement specific to the item indicating a normal medical finding for insertion into the itemised medical report;
- receiving from the user a dictated text comment (510b) for linking with a user-selected item (210b) of the checklist;
- replacing the default statement (510a"") linked to the user-selected item (210b) with the dictated text comment (510b);
- receiving an input indicating a user-selected sub-area (415) of the medical image (410);
- updating and generating for display in the display area (400) the checklist (205) wherein a thumbnail image (220) of the selected sub-area (415) is provided adjacent to or at least partially overlapping with the selected item (210b);
- generating an editable itemised medical report (600) containing items (210a', 210a", 210a'", 210a‴) of the checklist (205) and the default statement (510a', 510a", 510a'") linked to each item (210a', 210a", 210a'", 210a‴) not selected by the user for comment and dictated text comment (510b) linked to each item (210b) selected by the user, wherein the editable itemised medical report (600) also comprises one or more thumbnail image(s) of the selected sub-area(s) (415) associated with the selected item(s) (210b).

The steps of receiving and replacing may be optionally repeated for some of the other items (210a', 210a", 210a'") in the checklist.

The method may further providing the option of removing one or more items (210a', 210a", 210a'", 210a'", 210a'") from the checklist (205) before generating the editable itemised medical report (600).

The method may further comprise the steps of:
- receiving (from the user) an input of a selected item (210b) from the checklist (205) for linking with the dictated comment (510b); and
- generating for display in the display area (400) the checklist (205) updated with a visual marking indicating the selected item.

The selected sub-area (415) of the medical image (410) may be contextually related to the selected item (210b).

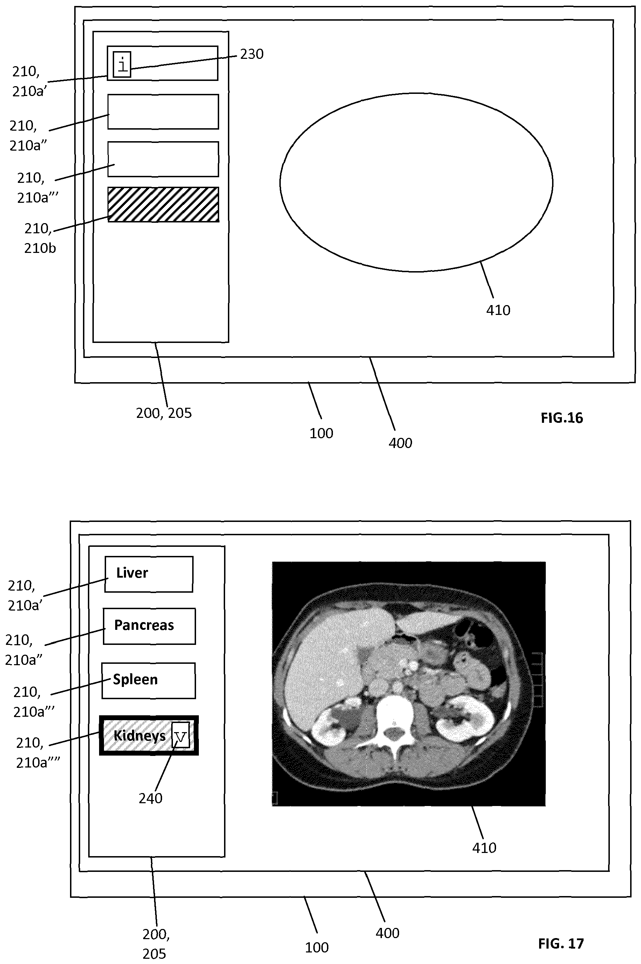

The method may further comprise the step of generating for display in the display area (400) a selectable information button (230 - FIG. 16) disposed adjacent to or at least partially overlapping with one or more of the one or more selectable items (210), wherein selection of the information button (230) generates for display in the display area (400) pre-defined reference information relevant to the item.

The method may further comprise the option to the user to link a tag (240) to a selected item in the checklist. The method may further comprising the option to the user to link a tag (240) to a selected item in the checklist, which tag is different from the dictated comment (510b), may be selectable from a pre-defined list or may be user-defined comment, and is linked to the selected item (210b).

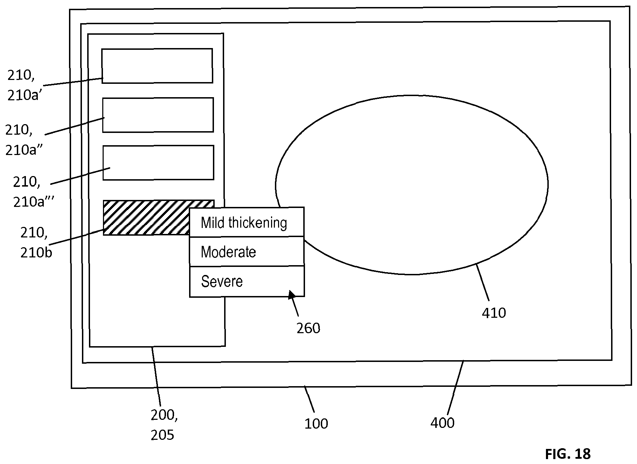

The method may further comprise the steps of
- generating for display in the display area (400) options (FIG. 18 - 260) for standardised reporting descriptions of abnormalities after selection of an item in the checklist (205),
- receiving an input (from the user) indicating a selection or rejection of the options
- adding the standardised reporting text linked to a selected option to the editable itemised medical report (600).

The sub-region (200) may be implemented as a user-moveable overlay.

The method may further comprise the steps (prior to the above steps) of:
- generating for display in the display area (400) a set of checklists containing two or more selectable checklists;
- receiving an input of a selected checklist from the set of checklists wherein the selected checklist (205) (being a personalised checklist) is that displayed in the sub-region (200).

The method may further comprise the steps of generating at least one of the selectable checklists in the set of the checklists using patient-specific information.

At least some of the items in the checklist (205) may be organised into groups. Items in the checklist that are organised into a group may be displayed and made available for receiving a dictated comment after the user has indicated a selection of that group.

Further described herein is a computer program or a computer program product adapted to perform a method as described herein, comprising a computer-readable data carrier comprising said computer program or computer program product.

### Legends to the figures

**FIG.1** shows a medical image displayed in a display area of single computer display unit, together with a checklist of items.
**FIG.2** shows mouse rolled over a checklist item to reveal a hidden default statement.
**FIG.** 3 shows a checklist of items provided with a selectable "remove" button (X).
**FIG.** 4 shows a selected checklist item disposed with a different marking.
**FIG.** 5 shows a format of a storage medium where items of the checklist are each linked to a default statement, or a default statement replaced with user comment (dictated text).
**FIG.** 6 shows a layout of an editable itemised medical report generated by the present method.
**FIG.** 7 shows an exemplary medical image displayed in a display area of single computer display unit, together with a checklist of items.
**FIG.** 8 shows an exemplary editable itemised medical report generated based on a dictated comment in respect of "Kidney" as a selected item.
**FIG.** 9 shows an example not of the invention, where the radiologist uses a two separate computer screens, a left screen to display a long-form editable template for the medical report and a right screen to view a radiological image.
**FIG. 10** shows an example not of the invention, where the radiologist uses a one computer screen to display a long-form editable template for the report and a radiological image.
**FIG. 11** shows a work flow of the art for generating a medical report
**FIG. 12** shows a work flow of the present invention for generating a medical report
**FIG. 13** shows an example where the radiologist uses two computer screens, one computer screen (right hand) to display the radiological image and the checklist, and a second computer screen (left hand) to display the editable itemised medical report.
**FIG. 14** shows selection a sub-area of the medical image for copy/pasting or dragging into a corresponding item in the checklist.
**FIG. 15** shows selection a sub-area of the medical image for copy/pasting or dragging into a corresponding item in the checklist on the right screen, and its appearance in the editable itemised medical report in left screen.
**FIG. 16** shows a checklist where one item is provided with an "information" button.
**FIG.** 17 shows a checklist where one item is provided with a "variant" button.
**FIG. 18** shows a checklist where one item is provided with selectable option list.

### Detailed description of the invention

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of'.

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous. Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilised and structural or logical changes may be made without departing from the scope of the present invention. The following detailed description, therefore, is not to be taken in a limiting sense, and the scope of the present invention is defined by the appended claims.

The present invention relates to method for assisting a user in generating an itemised medical report from at least one medical image. The method may be computer implemented, or implemented in a system or apparatus.

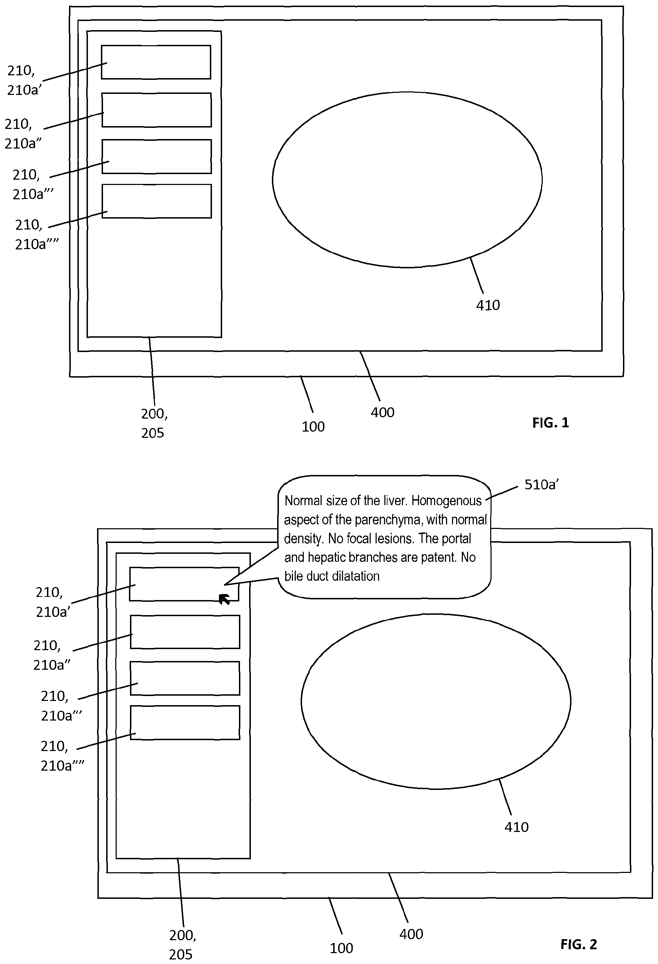

The at least one medical image (410) is displayed in a display area (400) of single computer display unit (100) as shown, for instance in **FIG. 1****.** In the same display area (400), a sub-region (200) containing a checklist (205) specific to the one or more images is displayed. This checklist comprises a list of selectable items (210), each item representing an organ, structure, or abnormality that has to be checked by the user. The user is typically a radiologist or trainee radiologist. As an example, for a CT scan of the abdomen, the items (210) in the checklist (205) may represent organs to be checked, for instance "liver", "pancreas", "spleen", "stomach", "kidneys", etc. It is noted that a single computer display unit (100) typically receives one set of data (e.g. image) signals through a single connecting cable. Where two computer display units are described herein, each display unit typically independently receives one set of data (*e*.*g*. image) signals through a single connecting cable i.e. there are two sets of data signals and two connecting cables in total.

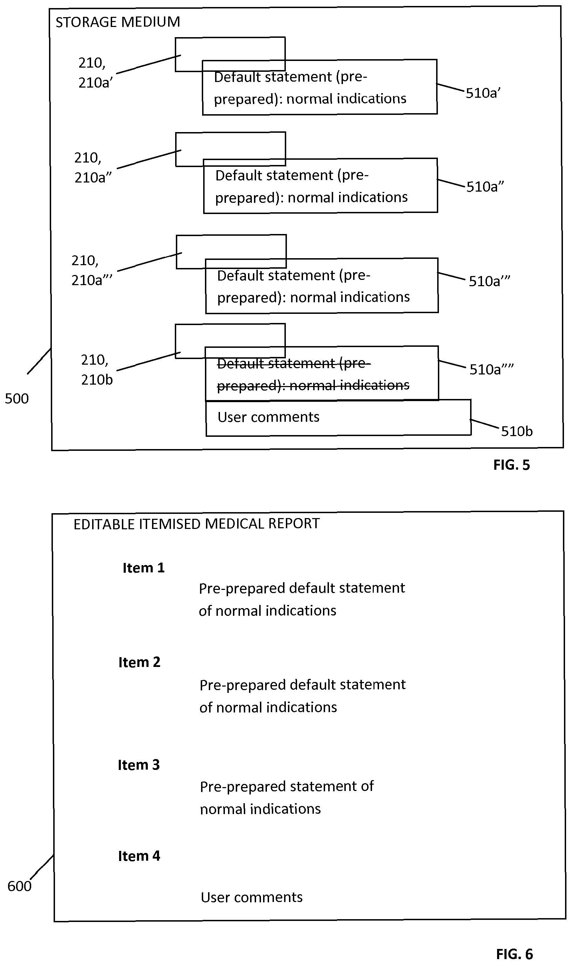

Linked to each item in the checklist is a default statement **(****FIG. 5**, 510a', 510a", 510a‴, 510aʺʺ) that is a statement indicative of the normality of the item. It is a standard medical finding (of normality) which does not need further annotations nor explanations by the user. It is a pre-prepared statement. The statement is stored in a database. The default statement (510a', 510a", 510a‴, 510aʺʺ) is not displayed by default as part of the checklist (205). The default statement (510a', 510a", 510a‴, 510aʺʺ) is visible in the editable itemised medical report (600) eventually generated. It is understood that if an item in the checklist is not user-selected for providing a comment, the default statement (510a', 510a", 510a'", 510aʺʺ) is intended to appear in the editable itemised medical report (600).

For the example of **FIG. 1****,** the default statement linked to "liver" may state, for instance: "Normal size of the liver. Homogeneous aspect of the parenchyma, with normal density. No focal lesions. The portal and hepatic branches are patent. No bile duct dilatation". The default statement indicates that the liver is normal. The default statement (510a') is hidden text linked to each item is not displayed by default in the image display area during routine workflow. The default statement (510a') may be displayed by a simple action of the user e.g., mouseover as shown in **FIG. 2****.**

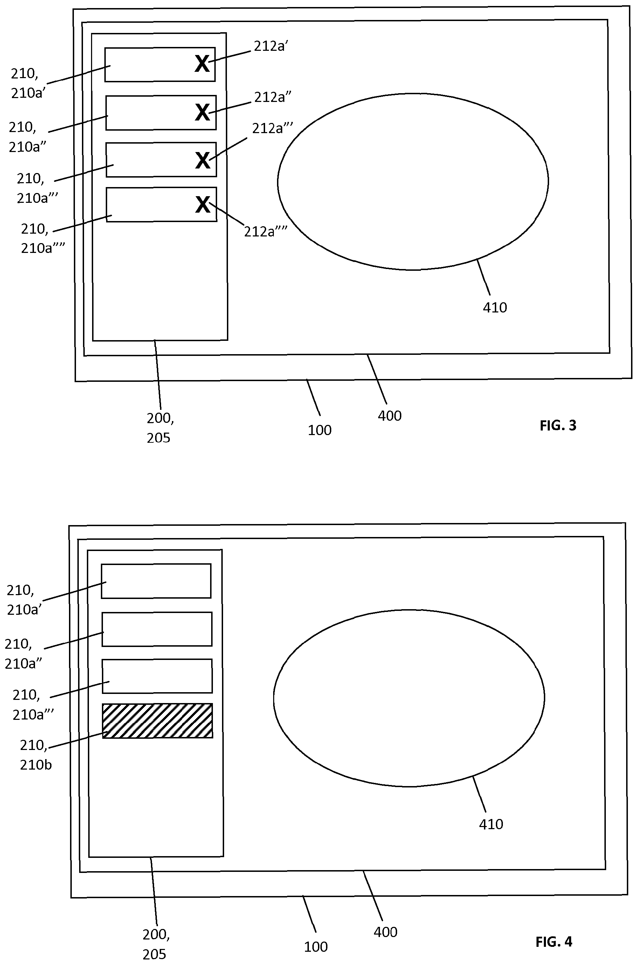

In some instances, the radiologist may not want to report on one or more items in the checklist (for example because the anatomical structure corresponding to an item in the checklist is not well seen on the images of a particular patient). In one embodiment of the invention, one or more item(s) can be removed from the checklist by a simple action (see figure). The selectable item (210) may be removable by providing and displaying next to the selectable item a "remove" icon which when selected removes the selectable item from display. For instance, in FIG. 3, each selectable item (210a', 210a", 210a‴, 210a"") is provided with an adjacent "remove" icon (212a', 212a", 212a‴, 212a"") that is a cross (X). When an item is selected (e.g. FIG. 4, 210b), for instance using a pointing device (e.g. mouse, digit), the item becomes active and the user can input user comments **(****FIG.** 5, 510b) related to the selected item (210b), for instance by dictation. The comments are typically visual observations pertinent to the medical image. The checklist (205) generated for display in the display area (400) may be updated with a visual marking (220) indicating the selected item (210b). The visual marking is a different marking from a non-selected item (e.g. 210a', 210a", 210a‴ in FIG. 4). The item may be selected before or after input of report information. The inputted user comments **(****FIG.** 5, 510b) are stored in a storage medium (500) and linked to selected item (210b); it replaces the default statement (510aʺʺ). The other items of the checklist (210a', 210a", 210a‴) remain linked to the default statement (510a', 510a", 510a‴) i.e. to pre-prepared statement indicative of the normality of the item **(****FIG.** 5).

An exemplary workflow using the invention is as follows.

While scrolling through the images, the radiologist may detect one or several abnormalities in the stack of images. During image viewing, the radiologist starts to dictate user comments about a specific item that appears abnormal. At the end of the dictation, he indicates (e.g. by speech or by clicking on the item) which is the corresponding item in the checklist (210b), and gives a command (e.g. via speech or using button on microphone) to paste the dictated text (user comments) to the text field linked to that item. Alternatively, the "abnormal" item in the checklist may be "activated" (selected) before the dictation starts. The checklist (205) generated for display in the display area (400) is now updated with a visual marking (220) indicating the selected item **(****FIG.** 4, 210b) with a different marking from a non-selected item (210a', 210a", 210a‴). Once an item has been selected, it may retain a distinguishing visual marking to indicate that a user comment has been provided thereon.

The dictated comment (510b) relevant for the selected item is stored and linked to the selected item (210b) in the checklist. The other items of the checklist (210a', 210a", 210a‴) remain linked to the respective default statements (510a', 510a", 510a‴) i.e. to the pre-prepared statement indicative of the normality of the item **(****FIG. 5****).**

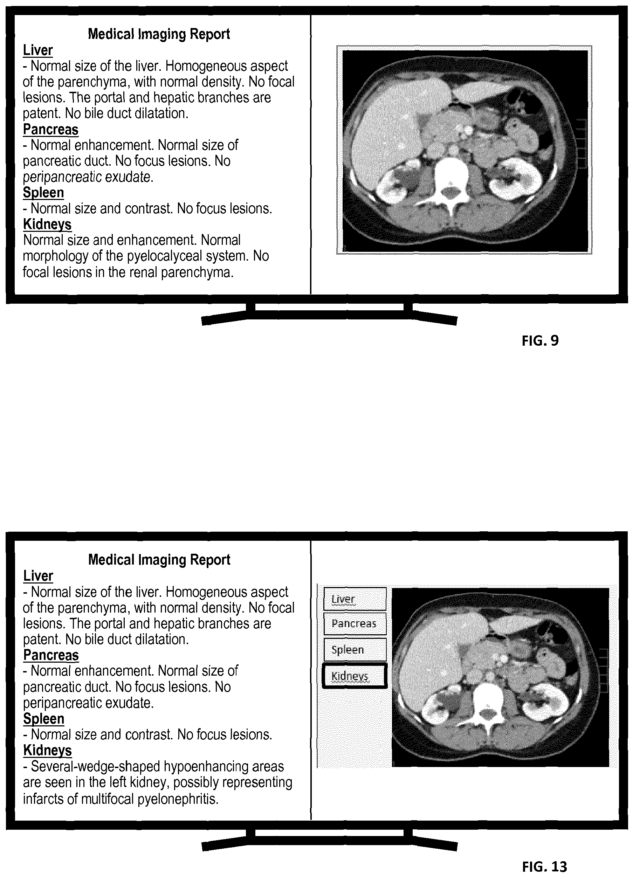

Although not displayed in the display area (100), the dictated comment (510b) is now linked to the selected item (210b), and will be used instead of the default statement (510aʺʺ) for the eventual itemised medical report **(****FIG. 6****,** 600). It is an aspect of the invention that the dictated comment (510b) is simultaneously displayed dynamically on a second computer display unit as shown, for instance, in **FIG. 13****.**

If needed the radiologist repeats the procedure described above for other items in the checklist.

Upon receiving an input from the radiologist or other user, an editable itemised medical report is generated **(****FIG. 6****,** 600). This report contains all items in the checklist (210a', 210a", 210a‴, 210b) except those removed by the radiologist. For each item that has not been selected by the user, the default statement (510a', 510a", 510a‴) is used for the report. For each item that has been selected by the user, the default statement (510aʺʺ) is replaced by the dictated user comments (510b) provided by the user. In the example of **FIG. 5****,** the medical imaging report would have the format as shown in **FIG. 6****.** For items 1 to 3, the default statement (510a', 510a", 510a‴) is used in the report. For Item 4, the dictated text (510b) is used.

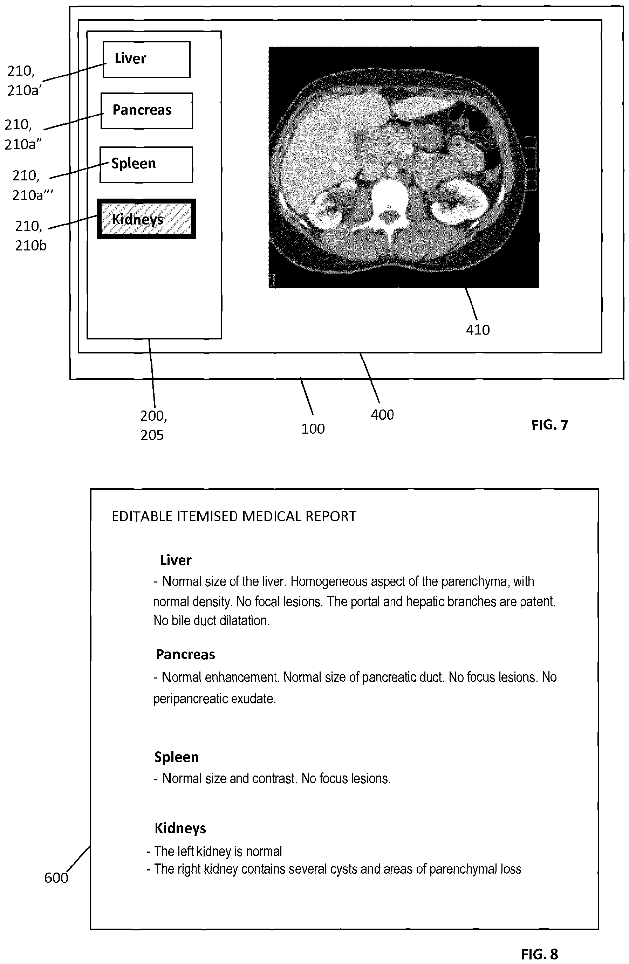

A more concrete example is given as follows, illustrated in **FIGs. 7** and **8****.** A radiologist has to make a report of a CT study of the upper abdomen. As a simplification, it is assumed that the report has to contain comments on the following four organs only: liver, pancreas, spleen, kidneys. According to the description above, the radiologist scrolls through the images and the 4 relevant organs are included as items in the checklist (see **FIG. 7****).** While the radiologist considers the liver, pancreas, and spleen to be normal, he detects an abnormality in one of the kidneys and wants to describe the abnormality using dictation. The radiologist selects the item "kidneys" **(****FIG. 7****,** 210b) either before or after the actual dictation and dictates the following sentence "The left kidney is normal. The right kidney contains several cysts and areas of parenchymal loss." In this example, the radiologist does not detect any further abnormalities. During the process or after receiving an input from the radiologist indicating that the dictation is finished, the method provides an editable itemised medical report as shown in **FIG. 8****.**

The invention provides a solution for the current lack of implementation of itemised reporting in clinical practice. The report generated according to the method of the invention is a well-structured itemised report. However, unlike in solutions described in previous art, this report can be generated with minimal distraction of the user (radiologist). The only distraction from the ideal cognitive workflow (consisting of combined image viewing and dictating) is the need to give an indication to which organ in the checklist the dictated text has to be linked. Moreover, the invention saves time for the radiologist: his dictation does not have to include a description of normal organs (these descriptions are automatically generated); as such he can focus on the dictation of what is abnormal.

The invention makes use of a checklist that is displayed within the same display area (400) as the medical images (410), thus reducing the "look away time" for the radiologist, and that, at the same time, represents an itemised report. There may be a reduction in oculomotor distress, such as headaches, eyestrain, blurred vision and general discomfort over time. While displaying a full itemised standard report within the same area as the images would not be practical for the radiologist, the checklist as described above allows to combine the best of both worlds: the advantages of itemised reporting can be obtained with minimal or no workflow interruption.

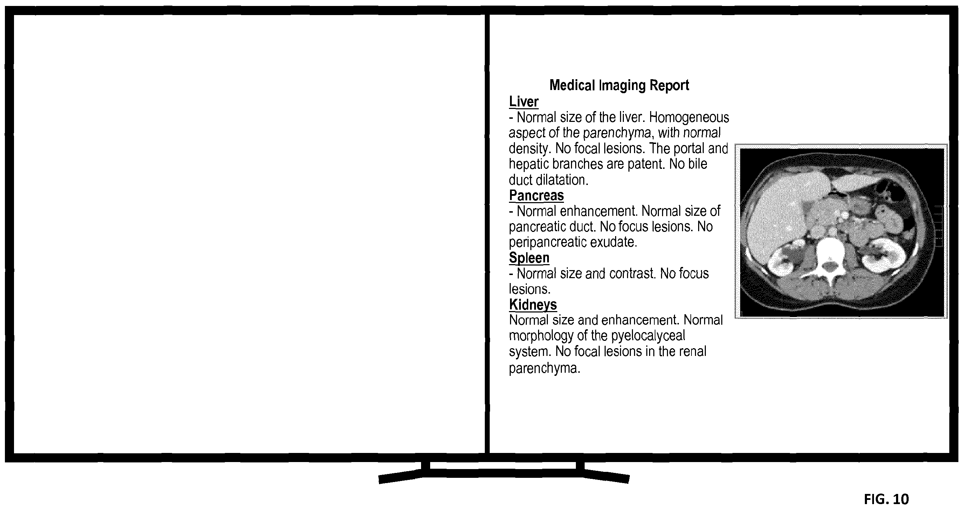

In order to appreciate the difference with the current art, the classic process to make an itemised report in the case described above will be illustrated and compared with the workflow according to the invention. As mentioned above, medical images and templates for reporting are classically displayed on separate display areas (see **FIG. 9****).** This is done to allow maximum magnification of the images, which may be required for detection of subtle abnormalities. As illustrated in **FIG. 10****,** displaying both the itemised report and the images in the same display would be impractical. Both the images and the text of the report would be displayed at suboptimal size, and the left screen would be left unused.

Moreover, in order to link dictated text to a specific item in the itemised report, the radiologist has to try to find the relevant paragraph in the report, which is obviously more complicated than finding the relevant item in a checklist.

It is an aspect of the invention that the checklist is displayed in the same display area as the medical images. Because of the nature of a checklists, which comprises a list of items rather than extensive text or paragraphs, the checklist does not significantly interfere with optimal magnification of the medical images. Moreover, when an abnormality is detected, the relevant item in the checklist can be readily identified.

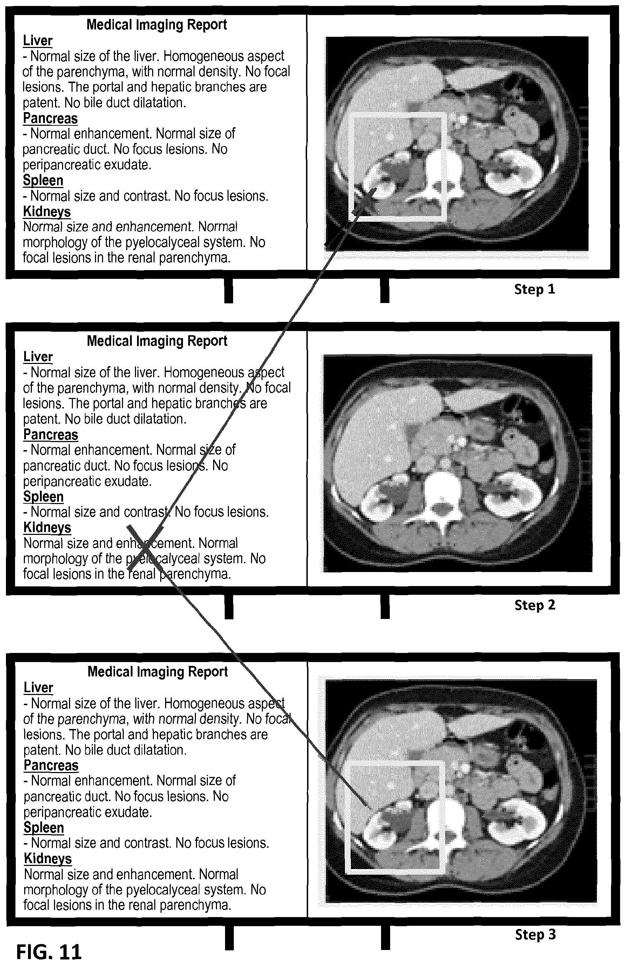

**FIG. 11** further illustrates differences between current art and the present method in terms of interruption of the cognitive process of the radiologist. **FIG. 11** illustrates the current art for standardized (or itemised) reporting. The radiologist starts with a template containing a normal report (left screen) and a screen displaying images (right screen). In step 1, the radiologist analyzes the images and detects an abnormality. In step two, the radiologist has to look away from the screen displaying the images and try to find the relevant paragraph in the standard report. Also in step 2, the radiologist has to indicate that he wants to dictate an observation in the standard report under the item "kidneys". In order to do so, he has to put the cursor of the dictation tool in the right place in the text field. Furthermore, he has to remove the standard text provided in the template. All this together represents a major workflow interruption. Finally, in step three, the radiologist describes the abnormality using dictation.

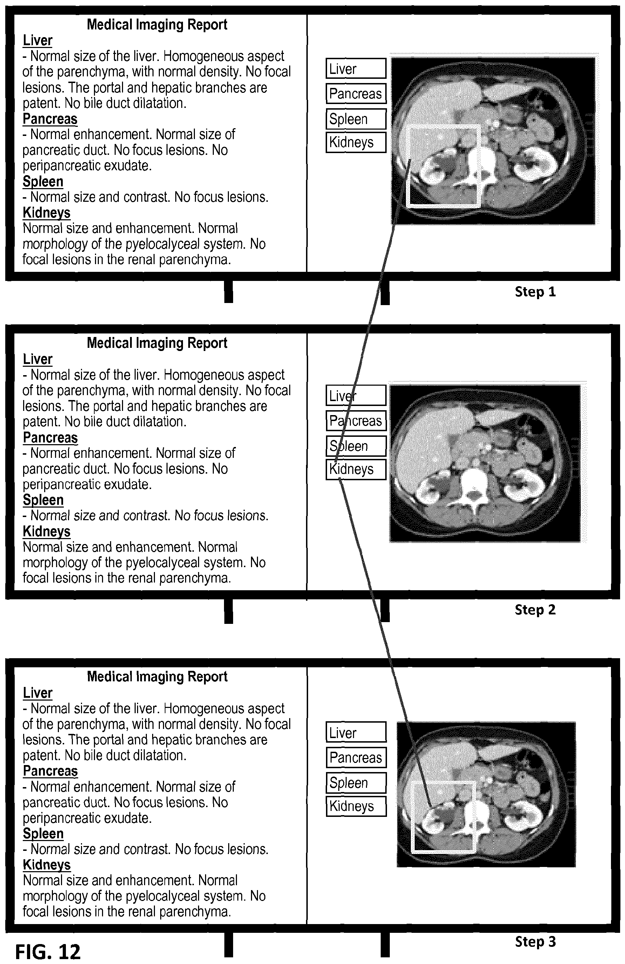

By comparison a workflow according to the present method is illustrated in **FIG. 12****.** Step 1 is identical as above. In step two, the radiologist simply clicks on the item "kidneys" in the checklist. This is immediately followed by the dictation (step 3). The workflow interruption is negligible.

In clinical practice, there is usually more than one abnormality to be reported. Modern CT and MRI scanners produce hundreds of images per exam and small anatomical structures are displayed with exquisite detail. It is not uncommon that five or ten variants or abnormalities are reported during viewing images of a single study. It is obvious that in such a situation the invention provides a huge advantage in terms of workflow optimization.

According to one embodiment of the invention, the default statement (510a', 510a", 510a‴, 510aʺʺ) can be displayed before dictation in a display area separate from the image display area (eg on a separate screen as illustrated below).

In a further embodiment of the invention, the editable itemised medical report is displayed outside of the display area (400) of single computer display unit (100) as shown, for instance in **FIG. 13****;** the editable itemised medical report is displayed is displayed on a second computer display unit. The editable itemised medical report may be updated during the reporting process, i.e., default statement is replaced by the text dictated by the radiologist in the report.

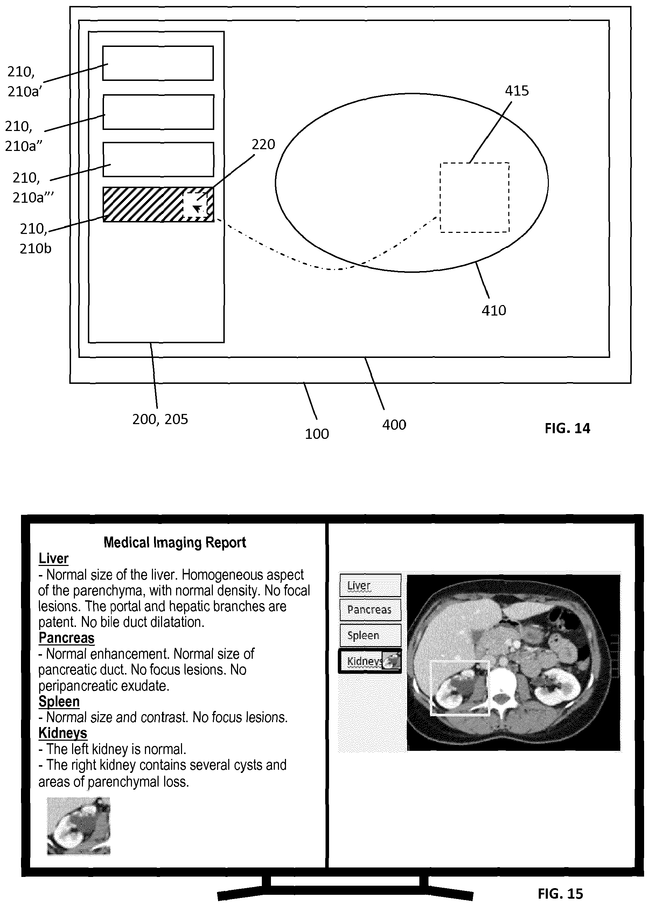

In a further embodiment of the invention, the radiologist can select a sub-area (415) of the medical image (410); the image part contained in the selected sub-area can be copy/pasted or dragged (220) into the corresponding item in the checklist (instead of just clicking on the item) as illustrated, for instance, in **FIG. 14****.** The selected sub-area (415) of the medical image (410) may be contextually related to the selected item (210b). As such, two functions are combined: (1) the item (in the checklist) is selected and (2) the selected image part is linked to the selected item. This allows the radiologist to produce an itemised report illustrated with relevant images showing the abnormalities that are described. The image can be displayed in the report adjacent to the corresponding dictation (see **FIG. 15****).** All this can be done without any additional workflow interruption for the radiologist.

Another embodiment of the invention further comprises the step of generating for display in the display area (400) a selectable information button (230 - **FIG. 16****)** disposed adjacent to or at least partially overlapping with one or more of the one or more selectable items (210), wherein selection of the information button (230) generates for display in the display area (400) pre-defined reference information relevant to the item.

The method may further comprise an option to the user to link a tag (240) to a selected item (210) in the checklist. A tag is a label that may be selectable from a pre-defined list, or a user-defined comment that may be dictated or entered using a manual-input device (e.g. keyboard, touch screen). It is different from the dictated comment (510b). A tag may be linked to the selected item (210), for example, by the method receiving (from the user) a selection of one from a plurality of labels. If the user does not take make any selection action, a default label may be assigned to the item (210) in the checklist. A tag may be linked to the selected item (210), for example, by the method receiving (from the user) a selection of one from three labels: "normal" (N), "anatomical variant" (V), or "pathologic" (P). If the user does not take make any selection action, the label "N" could be assigned by default to the item (210) in the checklist. The method may further comprise a step of generating for display in the display area (400) a selectable descriptive button (240) disposed adjacent to or at least partially overlapping with one or more of the one or more selectable items (210), wherein selection of the descriptive button (240) provides a label (i.e. a tag) to the item in the checklist. For instance in FIG. 17, a button "V" has been selected in relation the item "kidneys". This could mean that the dictation provided by the radiologist contains a description of an anatomical Variant.

Another embodiment of the invention further comprises the step of generating for display in the display area (400) a selectable descriptive button (240) disposed adjacent to or at least partially overlapping with one or more of the one or more selectable items (210), wherein selection of the descriptive button (240) provides a label to the item in the checklist. For instance in **FIG. 17****,** a button "V" has been selected in relation the item "kidneys". This could mean that the dictation provided by the radiologist contains a description of an anatomical Variant.

In a further embodiment of the invention, after selection of an item in the checklist (*e*.*g*. **FIG. 18****,** 210b), one or more descriptive terms are displayed and may be selected by the user (see **FIG. 18****,** 260). Selection of a descriptive term automatically generates a corresponding text in the draft report. In the example below, the item in the checklist could be, for instance, "achilles tendon", and selection of the button displaying "moderate" would generate a standard text "There is moderate thickening of the Achilles tendon" in the report. Accordingly the method may further comprise the steps of
- displaying options (260) for standardised reporting descriptions of abnormalities after selection of an item in the checklist
- receiving an input from the user indicating whether or not to choose one or these options
- adding the standardised reporting text linked to a selected item (if any) to the editable draft report

A computing device or system may also be provided configured for performing the method as described herein. The system may comprise circuitry configured performing the method as described herein. Typically the circuitry comprises a processor and a memory.

A computer program is also provided having instructions which when executed by a computing device or system cause the computing device or system to perform the method as described herein.

A computer readable medium may also be provided having stored thereon a computer program as described herein.

A data stream which is representative of a computer may also be provided.

## Claims

1. A method for assisting a user in generating an itemised medical report from at least one medical image (410), comprising the steps of:
- generating for display and displaying in a display area (400) of single computer display unit (100) at least one of the medical images (410);
- generating for display and displaying in the same display area (400), a sub-region (200) containing a checklist (205) specific to the one or more images, comprising a list of selectable items (210), wherein each item (210a', 210a", 210a'", 210a'", 210a‴) in the checklist is linked to a default statement (510a' , 510a", 510a‴, 510aʺʺ) which default statement is by default not displayed in the same display area (400) and is a pre-prepared statement specific to the item indicating a normal medical finding for insertion into the itemised medical report;
- receiving from the user a dictated text comment (510b) for linking with a user-selected item (210b) of the checklist;
- replacing the default statement (510a"") linked to the user-selected item (210b) with the dictated text comment (510b);
- receiving an input indicating a user-selected sub-area (415) of the medical image (410);
- updating and generating for display in the display area (400) the checklist (205) wherein a thumbnail image (220) of the selected sub-area (415) is provided adjacent to or at least partially overlapping with the selected item (210b); and
- generating an editable itemised medical report (600) containing items (210a', 210a", 210a'", 210a‴) of the checklist (205), and
- the default statement (510a', 510a", 510a'") linked to each item (210a', 210a", 210a'", 210a'") not selected by the user for comment, and
- dictated text comment (510b) linked to each item (210b) selected by the user,
wherein
- the editable itemised medical report (600) also comprises one or more thumbnail image(s) of the selected sub-area(s) (415) associated with the selected item(s) (210b).

2. Method according to claim 1, further providing the option of removing one or more items (210a', 210a", 210a'", 210a'", 210a'") from the checklist (205) before generating the editable itemised medical report (600).

3. Method according to claim 1 or 2 further comprising the steps of
- receiving an input of a selected item (210b) from the checklist (205) for linking with the dictated comment (510b); and
- generating for display in the display area (400) the checklist (205) updated with a visual marking indicating the selected item.

4. The method according to any of claims 1 to 3 further comprising the step of generating for display in the display area (400) a selectable information button (230 - FIG. 16) disposed adjacent to or at least partially overlapping with one or more of the one or more selectable items (210), wherein selection of the information button (230) generates for display in the display area (400) pre-defined reference information relevant to the item.

5. The method according to any of claims 1 to 4 further comprising the option to the user to link a tag (240) to a selected item in the checklist, which tag is different from the dictated comment (510b), may be selectable from a pre-defined list or may be user-defined comment, and is linked to the selected item (210b).

6. The method according to any of claims 1 to 5 further comprising the steps of
- generating for display in the display area (400) options (FIG. 18 - 260) for standardised reporting descriptions of abnormalities after selection of an item in the checklist (205);
- receiving an input indicating a selection or rejection of the options; and
- adding the standardised reporting text linked to a selected option to the editable itemised medical report (600).

7. The method according to any of claims 1 to 6 wherein the sub-region (200) is implemented as a user-moveable overlay.

8. The method according to any of claims 1 to 7 further comprising the steps of:
- generating for display in the display area (400) a set of checklists containing two or more selectable checklists; and
- receiving an input of a selected checklist from the set of checklists wherein the selected checklist (205) is that displayed in the sub-region (200).

9. The method according to claim 8, further comprising the step of generating at least one of the selectable checklists in the set of the checklists using patient-specific information.

10. The method according any of claims 1 to 9, wherein at least some of the items in the checklist (205) are organised into groups.

11. The method according claim 10 wherein items in the checklist that are organised into a group are displayed and made available for receiving a dictated comment after the user has indicated a selection of that group

12. A computer program having instructions which when executed by a computing device or system cause the computing device or system to perform the method of any one of claims 1 to 11.

## Patentansprüche

1. Verfahren zum Unterstützen eines Anwenders beim Erzeugen eines detaillierten medizinischen Berichts aus mindestens einem medizinischen Bild (410), das die folgenden Schritte umfasst:
- Erzeugen zur Anzeige und Anzeigen in einem Anzeigebereich (400) einer einzelnen Computeranzeigeeinheit (100) mindestens eines der medizinischen Bilder (410);
- Erzeugen zur Anzeige und Anzeigen in demselben Anzeigebereich (400) eines Teilbereichs (200), der eine Checkliste (205) enthält, die für das eine oder die mehreren Bilder spezifisch ist, die eine Liste auswählbarer Elemente (210) umfasst, wobei jedes Element (210a', 201a", 210a‴, 210a‴, 210a‴) in der Checkliste auf eine Standardaussage (510a', 510a", 510a‴, 510aʺʺ) verlinkt ist, wobei die Standardaussage standardmäßig nicht in demselben Anzeigebereich (400) angezeigt wird und eine vorab vorbereitete Aussage ist, die für das Element spezifisch ist, die einen üblichen medizinischen Befund zum Einsetzen in den detaillierten medizinischen Bericht angibt;
- Empfangen eines diktierten Textkommentars (510b) zum Linken mit einem vom Anwender ausgewählten Element (210b) der Checkliste vom Anwender;
- Ersetzen der Standardaussage (510aʺʺ), die mit dem vom Anwender ausgewählten Element (210b) verlinket ist, durch den diktierten Textkommentar (510b);
- Empfangen einer Eingabe, die einen vom Anwender ausgewählten Teilbereich (415) des medizinischen Bildes (410) angibt;
- Aktualisieren und Erzeugen zur Anzeige im Anzeigebereich (400) der Checkliste (205), wobei ein Miniaturbild (220) des ausgewählten Teilbereichs (415) benachbart zu dem ausgewählten Element (210b) oder dieses zumindest teilweise überlappend bereitgestellt wird; und
- Erzeugen eines editierbaren, detaillierten medizinischen Berichts (600), der Elemente (210a', 210a", 210a‴, 210a‴) der Checkliste (205) und
- die Standardaussage (510a', 510a", 510a‴), die mit jedem Element (210a', 210a", 210a‴, 210a‴), das nicht zur Kommentierung durch den Anwender ausgewählt ist, verlinkt ist, und
- einen diktierten Textkommentar (510b), der mit jedem Element (210b), das durch den Anwender ausgewählt ist, verlinkt ist, enthält, wobei
- der editierbare, detaillierten medizinische Bericht (600) außerdem ein oder mehrere Miniaturbilder des einen oder der mehreren ausgewählten Teilbereiche (415), die dem einen oder den mehreren ausgewählten Elementen (210b) zugeordnet sind, umfasst.

2. Verfahren nach Anspruch 1, das ferner die Wahlmöglichkeit des Entfernens eines oder mehrerer Elemente (210a', 210a", 210a‴, 210a‴, 210a‴) aus der Checkliste (205) vor dem Erzeugen des editierbaren, detaillierten medizinischen Berichts (600) bereitstellt.

3. Verfahren nach Anspruch 1 oder 2, das ferner die folgenden Schritte umfasst:
- Empfangen einer Eingabe eines ausgewählten Elements (210b) aus der Checkliste (205) zum Verlinken mit dem diktierten Kommentar (510b); und
- Erzeugen zur Anzeige im Anzeigebereich (400) der Checkliste (205), die mit einer visuellen Kennzeichnung, die das ausgewählte Element angibt, aktualisiert ist.

4. Verfahren nach einem der Ansprüche 1 bis 3, das ferner den Schritt des Erzeugens zur Anzeige im Anzeigebereich (400) einer auswählbaren Informationsschaltfläche (230 - Fig. 16), die benachbart zu einem oder mehreren des einen oder der mehreren auswählbaren Elemente (210) oder diese zumindest teilweise überlappend angeordnet ist, umfasst, wobei die Auswahl der Informationsschaltfläche (230) vorab definierte Referenzinformationen, die für das Element relevant sind, zur Anzeige im Anzeigebereich (400) erzeugt.

5. Verfahren nach einem der Ansprüche 1 bis 4, das ferner die Wahlmöglichkeit für den Anwender, einen Kennsatz (240) mit einem ausgewählten Element in der Checkliste zu verlinken, umfasst, wobei sich der Kennsatz von dem diktierten Kommentar (510b) unterscheidet, aus einer vorab definierten Liste auswählbar sein kann oder ein vom Anwender definierter Kommentar sein kann und mit dem ausgewählten Element (210b) verlinkt wird.

6. Verfahren nach einem der Ansprüche 1 bis 5, das ferner die folgenden Schritte umfasst:
- Erzeugen zur Anzeige im Anzeigebereich (400) von Wahlmöglichkeiten (Fig. 18 - 260) für standardisierte Berichtsbeschreibungen von Anomalien nach der Auswahl eines Elements in der Checkliste (205);
- Empfangen einer Eingabe, die eine Auswahl oder Zurückweisung der Wahlmöglichkeiten angibt; und
- Hinzufügen des standardisierten Berichtstextes, der mit einer ausgewählten Wahlmöglichkeit verlinkt ist, zu dem editierbaren, detaillierten medizinischen Bericht (600).

7. Verfahren nach einem der Ansprüche 1 bis 6, wobei der Teilbereich (200) als eine durch den Anwender bewegliche Überlagerung implementiert ist.

8. Verfahren nach einem der Ansprüche 1 bis 7, das ferner die folgenden Schritte umfasst:
- Erzeugen zur Anzeige im Anzeigebereich (400) einer Gruppe von Checklisten, die zwei oder mehr auswählbare Checklisten enthält; und
- Empfangen einer Eingabe einer ausgewählten Checkliste aus der Gruppe von Checklisten, wobei die ausgewählte Checkliste (205) diejenige ist, die im Teilbereich (200) angezeigt wird.

9. Verfahren nach Anspruch 8, das ferner den Schritt des Erzeugens mindestens einer der auswählbaren Checklisten in der Gruppe von Checklisten unter Verwendung patientenspezifischer Informationen umfasst.

10. Verfahren nach einem der Ansprüche 1 bis 9, wobei zumindest einige der Elemente in der Checkliste (205) in Gruppen organisiert sind.

11. Verfahren nach Anspruch 10, wobei Elemente in der Checkliste, die in einer Gruppe organisiert sind, angezeigt und für das Empfangen eines diktierten Kommentars zur Verfügung gestellt werden, nachdem der Anwender eine Auswahl dieser Gruppe angegeben hat.

12. Computerprogramm, das Anweisungen haßt, die dann, wenn sie durch eine Rechenvorrichtung oder ein Rechensystem ausgeführt werden, bewirken, dass die Rechenvorrichtung oder das Rechensystem das Verfahren nach einem der Ansprüche 1 bis 11 durchführt.

## Revendications

1. Procédé pour aider un utilisateur à générer un rapport médical détaillé à partir d'au moins une image médicale (410), le procédé comprenant les étapes suivantes :
- générer pour affichage et afficher dans une zone d'affichage (400) d'une seule unité d'affichage d'ordinateur (100) au moins une des images médicales (410) ;
- générer pour affichage et afficher dans la même zone d'affichage (400), une sous-région (200) contenant une liste de contrôle (205) spécifique aux une ou plusieurs images, comprenant une liste d'éléments sélectionnables (210), chaque élément (210a', 210a", 210a‴, 210aʺʺ, 210a‴ʺ) de la liste de contrôle étant lié à une déclaration par défaut (510a', 510a", 510a‴, 510aʺʺ), laquelle déclaration par défaut n'est, par défaut, pas affichée dans la même zone d'affichage (400) et est une déclaration préparée à l'avance, spécifique à l'élément, indiquant un résultat médical normal à insérer dans le rapport médical détaillé ;
- recevoir de l'utilisateur un commentaire textuel dicté (510b) à lier à un élément sélectionné par l'utilisateur (210b) de la liste de contrôle ;
- remplacer la déclaration par défaut (510aʺʺ) liée à l'élément sélectionné par l'utilisateur (210b) par le commentaire textuel dicté (510b) ;
- recevoir une entrée indiquant une sous-zone (415) de l'image médicale (410) sélectionnée par l'utilisateur ;
- mettre à jour et générer pour affichage dans la zone d'affichage (400) la liste de contrôle (205) dans laquelle une image miniature (220) de la sous-zone sélectionnée (415) est fournie à côté à l'élément sélectionné (210b) ou en le recouvrant au moins partiellement ; et
- générer un rapport médical détaillé modifiable (600) contenant des éléments (210a', 210a", 210a‴, 210aʺʺ) de la liste de contrôle (205), et
- la déclaration par défaut (510a', 510a", 510a‴) liée à chaque élément (210a', 210a", 210a‴, 210aʺʺ) non sélectionné par l'utilisateur en vue d'un commentaire, et
- un commentaire textuel dicté (510b) lié à chaque élément (210b) sélectionné par l'utilisateur, où :
- le rapport médical détaillé modifiable (600) comprend également une ou plusieurs images miniatures de la ou des sous-zones sélectionnées (415) associées au un ou plusieurs éléments sélectionnés (210b).

2. Procédé selon la revendication 1, fournier en outre la possibilité de supprimer un ou plusieurs éléments (210a', 210a", 210a‴, 210aʺʺ, 210a‴ʺ) de la liste de contrôle (205) avant de générer le rapport médical détaillé modifiable (600).

3. Procédé selon la revendication 1 ou 2, comprenant en outre les étapes suivantes :
- recevoir une entrée d'un élément sélectionné (210b) de la liste de contrôle (205) pour se lier au commentaire dicté (510b) ; et
- générer, pour affichage dans la zone d'affichage (400), la liste de contrôle (205) mise à jour avec un marquage visuel indiquant l'élément sélectionné.

4. Procédé selon l'une quelconque des revendications 1 à 3 comprenant en outre l'étape de générer pour affichage dans la zone d'affichage (400) un bouton d'information sélectionnable (230 - **FIG.** 16) disposé à côté ou en le recouvrant au moins partiellement un ou plusieurs des un ou plusieurs éléments sélectionnables (210), où la sélection du bouton d'information (230) génère pour affichage dans la zone d'affichage (400) des informations de référence prédéfinies pertinentes pour l'élément.

5. Procédé selon l'une quelconque des revendications 1 à 4 comprenant en outre la possibilité pour l'utilisateur de lier une étiquette (240) à un élément sélectionné dans la liste de contrôle, laquelle étiquette est différente du commentaire dicté (510b), peut être sélectionnable à partir d'une liste prédéfinie ou peut être un commentaire défini par l'utilisateur, et est liée à l'élément sélectionné (210b).

6. Procédé selon l'une quelconque des revendications 1 à 5, comprenant en outre les étapes suivantes :
- générer, pour affichage dans la zone d'affichage (400), des options (FIG. 18 - 260) pour des descriptions de rapports standardisés d'anomalies après sélection d'un élément dans la liste de contrôle (205) ;
- recevoir une entrée indiquant la sélection ou le rejet des options ; et
- ajouter le texte de rapport standardisé lié à une option sélectionnée au rapport médical détaillé modifiable (600).

7. Procédé selon l'une quelconque des revendications 1 à 6, dans lequel la sous-région (200) est mise en oeuvre sous la forme d'une superposition déplaçable par l'utilisateur.

8. Procédé selon l'une quelconque des revendications 1 à 7, comprenant en outre les étapes suivantes :
- générer, pour affichage dans la zone d'affichage (400), un ensemble de listes de contrôle contenant au moins deux listes de contrôle sélectionnables ; et
- recevoir une entrée d'une liste de contrôle sélectionnée dans l'ensemble de listes de contrôle, la liste de contrôle sélectionnée (205) étant celle affichée dans la sous-région (200).

9. Procédé selon la revendication 8, comprenant en outre l'étape de générer au moins une des listes de contrôle sélectionnables dans l'ensemble des listes de contrôle en utilisant des informations spécifiques au patient.

10. Procédé selon l'une quelconque des revendications 1 à 9, dans lequel au moins certains des éléments de la liste de contrôle (205) sont organisés en groupes.

11. Procédé selon la revendication 10, dans lequel les éléments de la liste de contrôle qui sont organisés en groupe sont affichés et rendus disponibles pour recevoir un commentaire dicté après que l'utilisateur a indiqué une sélection de ce groupe.

12. Programme d'ordinateur ayant des instructions qui, lorsqu'elles sont exécutées par un dispositif ou un système informatique, amènent le dispositif ou le système informatique à exécuter le procédé selon l'une quelconque des revendications 1 à 11.
